# EUROPEAN PATENT APPLICATION

(11) **EP 4 111 998 A1**
(43) Date of publication of application: **04.01.2023**
(21) Application number: 20922772.7
(22) Date of filing: 02.03.2020
(51) Int. Cl.: A61B 34/30, A61B 90/50

(54) **SURGICAL ROBOT**

(71) Applicant: Riverfield Inc., Tokyo 107-0052 (JP)
(72) Inventor: TADANO, Kotaro, Tokyo 160-0017 (JP); YAMAMOTO, Nobuaki, Tokyo 160-0017 (JP)
(74) Representative: adares Patent- und Rechtsanwälte Reininger & Partner GmbB
(86) International application number: PCT/JP2020/008744
(87) International publication number: WO 2021/176531

(57) **Abstract**

An object is to provide a surgical robot having a vertical drive mechanism that enables the up-down movement and capable of easily moving in accordance with the change in a surgical procedure or a diseased site.

The surgical robot of the present invention includes: a main body portion; an arm unit provided on the main body portion to hold an endoscope; an attachment member for detachably attaching the main body portion to a table portion of a bed; and a vertical drive mechanism that allows the main body portion to move along a first direction perpendicular to the table portion. In the surgical robot, at least the arm unit is pneumatically driven.

## Description

### [Technical Field]

The present invention relates to a surgical robot comprising: an arm unit that holds an endoscope; and a vertical drive mechanism.

### [Background Art]

The medical robot system described in Patent Document 1 includes a surgical robot fixed at a certain position, a movable bed, and a diagnostic imaging device, and the surgical robot has a plurality of arms each having a tip to which a manipulator and an endoscope are attached. The movable bed is configured such that the position, orientation, and posture can be changed with respect to a fixed station.

### [Prior Art Documents]

### [Patent Documents]

[Patent Document 1] JP2012-5557A

### [Summary of the Invention]

### [Problems to be solved by the Invention]

In the medical robot system described in Patent Document 1, however, although the movable bed can be moved, it is difficult to freely and quickly change the positional relationship between the manipulator or treatment tool and the patient in accordance with the case or the like and it is not preferred to change the position of the bed or the like due to the change in the up-down positional relationship between the manipulator or treatment tool and the patient because it imposes a burden on the patient.

On the other hand, if the bed is fixed and the surgical robot is moved up and down, a device with the large driving force is required to drive the surgical robot because conventional surgical robots are heavy. Accordingly, the weight including the surgical robot will be further increased, and it is therefore necessary to place the robot on the ground. Thus, even if the up-down movement becomes possible, it is difficult to move the robot in the horizontal direction when the surgical procedure is changed or a different diseased site is treated.

Furthermore, in order for the surgical robot to perform large stroke movements such as insertion of an endoscope into the patient's body, complicated driving is required through the joints of arms, which may further increase the weight of the drive mechanism.

An object of the present invention is therefore to provide a surgical robot having a vertical drive mechanism that enables the up-down movement and capable of easily moving in accordance with the change in a surgical procedure or a diseased site. Another object of the present invention is to provide a surgical robot that can easily and highly accurately respond even to large stroke movements by coordinating and driving a main body portion and an arm unit that extends from the main body portion.

### [Means for solving the Problems]

To achieve the above objects, the present invention provides a surgical robot comprising: a main body portion; an arm unit provided on the main body portion to hold an endoscope; an attachment member for detachably attaching the main body portion to a table portion of a bed; and a vertical drive mechanism that allows the main body portion to move along a first direction perpendicular to the table portion. In the surgical robot, at least the arm unit is pneumatically driven.

The surgical robot of the present invention preferably comprises: an arm drive control unit for driving the arm unit; a main body drive control unit for moving the main body portion in the first direction; and a cooperative drive control unit that coordinates the arm unit and the main body portion to allow the arm unit to perform a predetermined operation.

In the surgical robot of the present invention, the attachment member is preferably configured to be movable with respect to the table portion of the bed along a second direction different from the first direction, the surgical robot preferably includes a restriction member that restricts the attachment member from moving in the second direction with respect to the table portion of the bed, and the attachment member is preferably configured to be movable along the second direction when restriction by the restriction member is released.

The surgical robot of the present invention preferably comprises a fixing member that restricts the main body portion from moving along the first direction.

In the surgical robot of the present invention, the cooperative drive control unit is preferably configured to evacuate the arm unit to a predetermined position and fix the arm unit at the predetermined position.

In the surgical robot of the present invention, the vertical drive mechanism is preferably a ball screw provided on the main body portion, and the attachment member is preferably fixed to a nut portion that is displaced by rotation of a screw shaft of the ball screw.

The surgical robot of the present invention preferably comprises a fixing member that restricts the main body portion from moving along the first direction, and the fixing member is preferably a brake that restricts the screw shaft of the ball screw from rotating.

The surgical robot of the present invention preferably comprises a main body drive control unit for moving the main body portion in the first direction, and the brake preferably operates when a control signal from the main body drive control unit to a motor for driving the ball screw is absent for a predetermined time.

In the surgical robot of the present invention, the attachment member preferably comprises: an engagement portion that detachably engages the main body portion with the table portion of the bed; and a hold plate that is supported so as to be relatively movable on the engagement portion, and a support portion arranged around the table portion is preferably clamped by the engagement portion and the hold plate thereby to attach the main body portion to the table portion.

In the surgical robot of the present invention, the hold plate preferably includes a protrusion that projects toward the support portion side, and the protrusion and the support portion preferably come into contact with each other thereby to restrict the surgical robot from moving in the first direction.

### [Effect of the Invention]

According to the present invention, it is possible to provide a surgical robot having a vertical drive mechanism that enables the up-down movement and capable of easily moving in accordance with the change in a surgical procedure or a diseased site. Moreover, it is possible to provide a safe and convenient surgical robot that can easily and highly accurately respond even to large stroke movements, such as insertion of an endoscope, by coordinating and driving the arm unit and the a main body portion.

### [Brief Description of Drawings]

FIG. 1 is a perspective view illustrating a state in which a surgical robot according to an embodiment of the present invention is attached to a table portion of a bed.
FIG. 2 is an enlarged perspective view illustrating an arm unit of the surgical robot according to an embodiment of the present invention.
FIG. 3 is an enlarged perspective view illustrating a main body portion of the surgical robot according to an embodiment of the present invention.
FIG. 4 is a plan view illustrating the configuration of a bed to which the surgical robot according to an embodiment of the present invention is attached.
FIG. 5 is an enlarged perspective view, as viewed from above, illustrating an attachment portion between the main body portion and the bed according to an embodiment of the present invention.
FIG. 6 is an enlarged side view illustrating an engagement portion between the main body portion and a support portion.
FIG. 7 is an enlarged side view illustrating the engagement portion between the main body portion and the support portion and is also a diagram for describing insertion of a fixing pin.
FIG. 8 is a functional block diagram relating to a coordinated drive of the arm unit and a ball screw.

### [Embodiments for Carrying out the Invention]

Hereinafter, the surgical robot according to one or more embodiments of the present invention will be described in detail with reference to the drawings. The present embodiment exemplifies a surgical robot having an arm unit 20 that holds an endoscope 53 used for endoscopic surgery. In each figure, X-Y-Z coordinates are illustrated as reference coordinates. In the following description, the vertical direction will be referred to as a Z1-Z2 direction (up-down direction), and the left-right direction and the front-rear direction, which are orthogonal to each other in the horizontal plane orthogonal to the vertical direction, will be referred to as an X1-X2 direction and a Y1-Y2 direction, respectively. In the front-rear direction (Y1-Y2 direction), the Y1 side will be referred to as a rear side or a far side while the Y2 side will be referred to as a front side or a near side, and a state in which the lower side (Z2 side) is viewed from the upper side (Z1 side) may be referred to as a plan view.

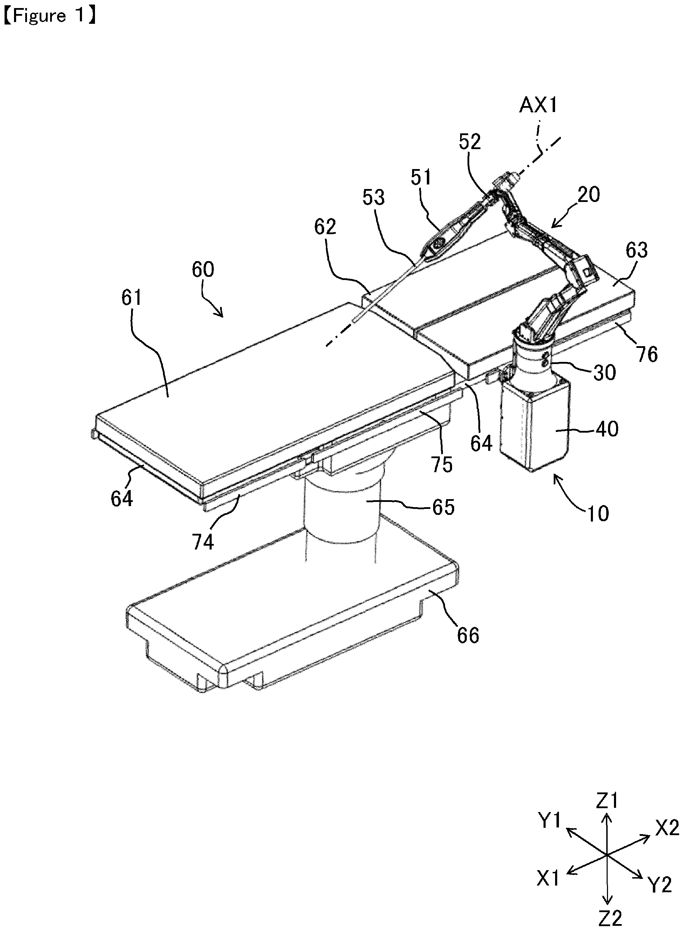

As illustrated in FIG. 1, the surgical robot (referred simply to as a "robot 10," hereinafter) includes the arm unit 20, a pedestal unit 30 that supports the arm unit 20, and a base unit 40 on which the pedestal unit 30 is provided. The arm unit 20, which holds an endoscope, is provided on a main body portion that is composed of the pedestal unit 30 and the base unit 40. The robot 10 is attached so that the base unit 40 can be detachably attached to any of table portions 61, 62, and 63 of a bed 60, on which a patient to be operated lies, and is movable in a direction along the outer circumference of the table portion.

In the example illustrated in FIG. 1, the arm unit 20 holds a camera head adapter 51 via a holder 52, and the camera head adapter 51 holds the endoscope 53. The holding form for the endoscope 53 by the arm unit 20 can be freely changed in accordance with the form, use, and the like of the endoscope 53 and is not limited to that illustrated in FIG. 1.

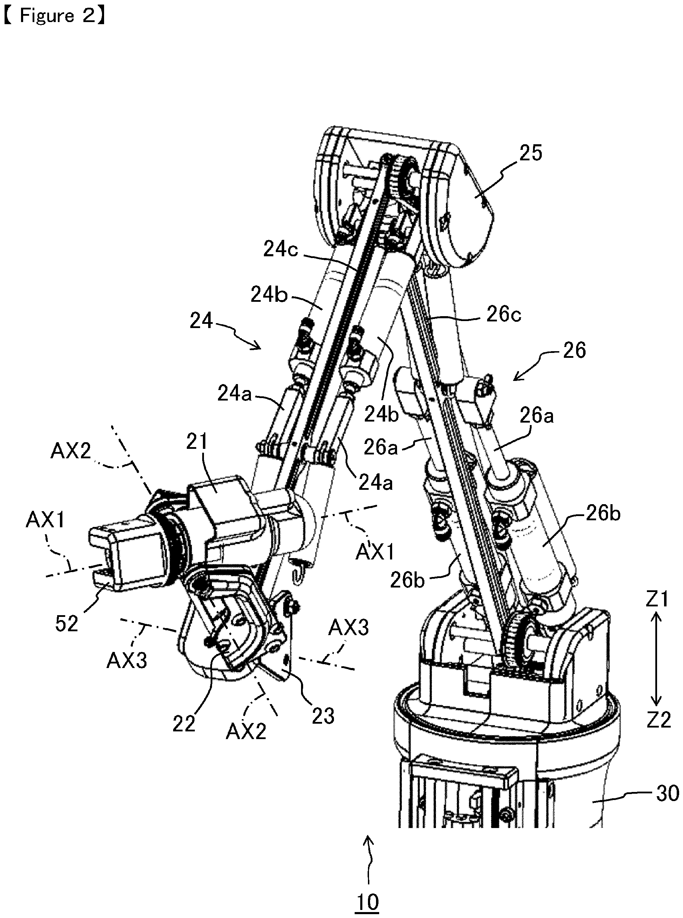

As illustrated in FIG. 2, the arm unit 20 includes a tip holding portion 21, a gimbal mechanism portion 22, a first joint portion 23, a first arm 24, a second joint portion 25, and a second arm 26 in this order from the holder 52 side to the pedestal unit 30 side. The holder 52 is attached to the tip holding portion 21.

The tip holding portion 21 is laterally supported by the gimbal mechanism portion 22 and detachably holds the holder 52 on the tip side thereby to hold the camera head adapter 51 attached to the holder 52.

The gimbal mechanism portion 22 supports the endoscope 53, which is held by the camera head adapter 51, in a rotatable manner about a first axis of rotation AX1 along an observation optical axis of the endoscope 53 and also supports the endoscope 53 in a rotatable manner about a direction intersecting with the first axis of rotation AX1, preferably about a second axis of rotation AX2 extending in a direction orthogonal to the first axis of rotation AX1.

The first joint portion 23 supports the gimbal mechanism portion 22 in a relatively rotatable manner about a third axis of rotation AX3 that extends so as to intersect with, more preferably so as to be orthogonal to, a plane including the first axis of rotation AX1 and the second axis of rotation AX2.

The first arm 24 forms a parallel link mechanism together with the first joint portion 23 and the second joint portion 25 and allows the first joint portion 23 and the second joint portion 25 to relatively move while maintaining their relative postures. The first arm 24 includes a pair of first rods 24a, a pair of first pneumatic actuator portions 24b, and a first self-weight compensation portion 24c.

The first rods 24a are a pair of rod-shaped members, one end portions of which are attached to the first joint portion 23 in a rotatable manner and the other end portions of which are attached to the second joint portion 25 in a rotatable manner.

The first pneumatic actuator portions 24b move the relative positions of the first joint portion 23 and the second joint portion 25. For example, each first pneumatic actuator portion 24b is an actuator including a cylinder and a piston and slides and drives the piston by receiving the supply of air with increased pressure. One end portion of the first pneumatic actuator portion 24b is attached in a rotatable manner to or in the vicinity of a position to which one first rod 24a is attached in the second joint portion 25, and the other end portion is attached in a rotatable manner to the middle of the other first rod 24a.

The first self-weight compensation portion 24c applies the force for moving the first joint portion 23 upward and is composed, for example, of a tension spring. One end portion of the first self-weight compensation portion 24c is attached to or in the vicinity of a position to which one first rod 24a is attached in the first joint portion 23, and the other end portion is attached to the middle of the other first rod 24a.

The second arm 26 forms a parallel link mechanism together with the second joint portion 25 and the pedestal unit 30 and allows the second joint portion 25 and the pedestal unit 30 to relatively move while maintaining their relative postures. The second arm 26 includes a pair of second rods 26a, a pair of second pneumatic actuator portions 26b, and a second self-weight compensation portion 26c.

The second rods 26a are a pair of rod-shaped members, one end portions of which are attached to the second joint portion 25 in a rotatable manner and the other end portions of which are attached to the pedestal unit 30 in a rotatable manner.

The second pneumatic actuator portions 26b move the relative positions of the second joint portion 25 and the pedestal unit 30. For example, each second pneumatic actuator portion 26b is an actuator including a cylinder and a piston and slides and drives the piston by receiving the supply of air with increased pressure. One end portion of the second pneumatic actuator portion 26b is attached in a rotatable manner to or in the vicinity of a position to which one second rod 26a is attached in the pedestal unit 30, and the other end portion is attached in a rotatable manner to the middle of the other second rod 26a.

The second self-weight compensation portion 26c applies the force for moving the second arm 26 in the vertical direction as a first direction and is composed, for example, of a tension spring. One end portion of the second self-weight compensation portion 26c is attached to or in the vicinity of a position to which one second rod 26a is attached in the pedestal unit 30, and the other end portion is attached to the middle of the other second rod 26a.

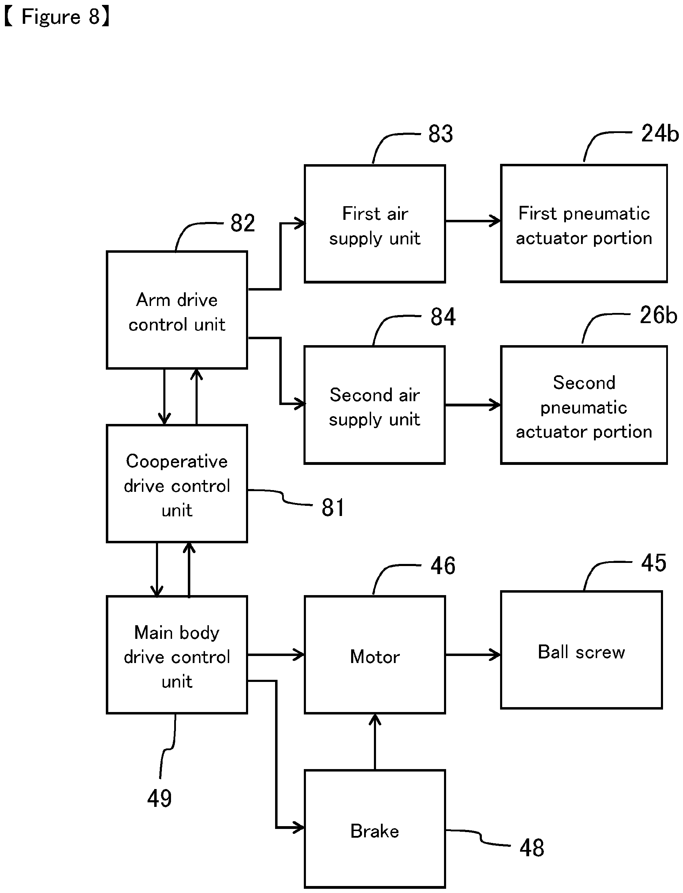

As illustrated in FIG. 8, the first pneumatic actuator portions 24b receive the supply of air with increased pressure from a first air supply unit 83, and the second pneumatic actuator portions 26b receive the supply of air with increased pressure from a second air supply unit 84. The first air supply unit 83 and the second air supply unit 84 supply the air of a predetermined pressure to the first pneumatic actuator portions 24b and the second pneumatic actuator portions 26b, respectively, at a predetermined time or cycle in accordance with control signals from an arm drive control unit 82. This allows the first arm 24 and the second arm 26 to be driven so that the arm unit 20 performs a predetermined drive, and the endoscope 53 can be placed at a predetermined position with a predetermined posture or moved from that position along a predetermined trajectory.

In the robot 10 having the above configuration, the arm unit 20 is operated by the pneumatic drive as will be described below.

The first pneumatic actuator portions 24b and the second pneumatic actuator portions 26b project the pistons from the cylinders or pull the pistons into the cylinders by being supplied with the driving air having an increased pressure. In the first arm 24, for example, when the pistons are projected from the cylinders to increase the longitudinal dimension of the first pneumatic actuator portions 24b, the first rods 24a of the first arm 24 rotate so that the first joint portion 23 moves upward. On the contrary, when the longitudinal dimension of the first pneumatic actuator portions 24b becomes shorter, the first rods 24a rotate so that the first joint portion 23 moves downward. The first self-weight compensation portion 24c is attached to the first arm 24 in a state of being stretched to increase the longitudinal dimension and therefore generates the biasing force in a direction in which the longitudinal dimension of the first self-weight compensation portion 24c becomes shorter. This biasing force acts as the force for the first joint portion 23 to rotate upward against the own weight of the endoscope 53 and the like.

By supporting the first joint portion 23 with the first arm 24 and the second arm 26 each having a parallel link, it is easy to keep constant the posture of the first joint portion 23 even when the arrangement position of the first joint portion 23 is changed.

By providing the first self-weight compensation portion 24c and the second self-weight compensation section 26c, it is possible to reduce the driving force required for the first pneumatic actuator portions 24b and the second pneumatic actuator portions 26b to move the position of the first joint portion 23 which supports the endoscope 53, as compared with the case of not providing the first self-weight compensation portion 24c or the second self-weight compensation portion 26c. Thus, the sizes of the first pneumatic actuator portions 24b and the second pneumatic actuator portions 26b can be easily reduced, and the weight saving of the robot 10 can be achieved.

By using the first pneumatic actuator portions 24b and the second pneumatic actuator portions 26b, it is easy to increase the weight-to-output ratio, and a simple linear motion operation can be achieved without using a deceleration mechanism. As a result, it becomes easier to reduce the size and weight of the robot 10.

The description will then be directed to the attachment to any of the table portions 61 to 63 of the bed 60 and a vertical drive mechanism for driving the robot 10 along the first direction perpendicular to the table portions 61 to 63 of the bed 60. In the following description, the first direction is a direction along the vertical direction because the description is made for an example in which the table portions 61 to 63 of the bed 60 are along the horizontal direction.

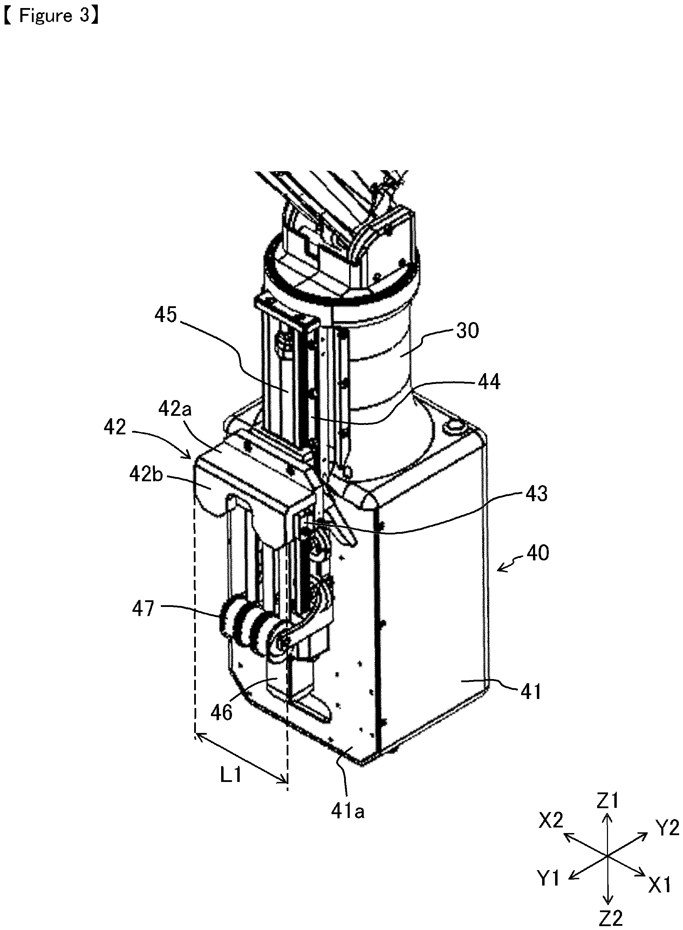

As illustrated in FIG. 3, the base unit 40 of the robot 10 includes an engagement portion 42 that is provided on a rear surface 41a on the rear side (Y1 side) of a housing portion 41 of the base unit 40 via a linear guide 44 and a ball screw 45 and further includes a hold plate 43 that is supported so as to be relatively movable on the engagement portion 42. The engagement portion 42 and the hold plate 43 constitute an attachment member for detachably attaching the base unit 40 (main body portion) to any of the table portions 61 to 63 of the bed 60. The engagement portion 42 includes an extending portion 42a that extends in a plate shape rearward from the rear surface 41a and a tip engagement portion 42b that extend in a plate shape downward from the rear tip of the extending portion 42a. The engagement portion 42 has a length L1 in the left-right direction.

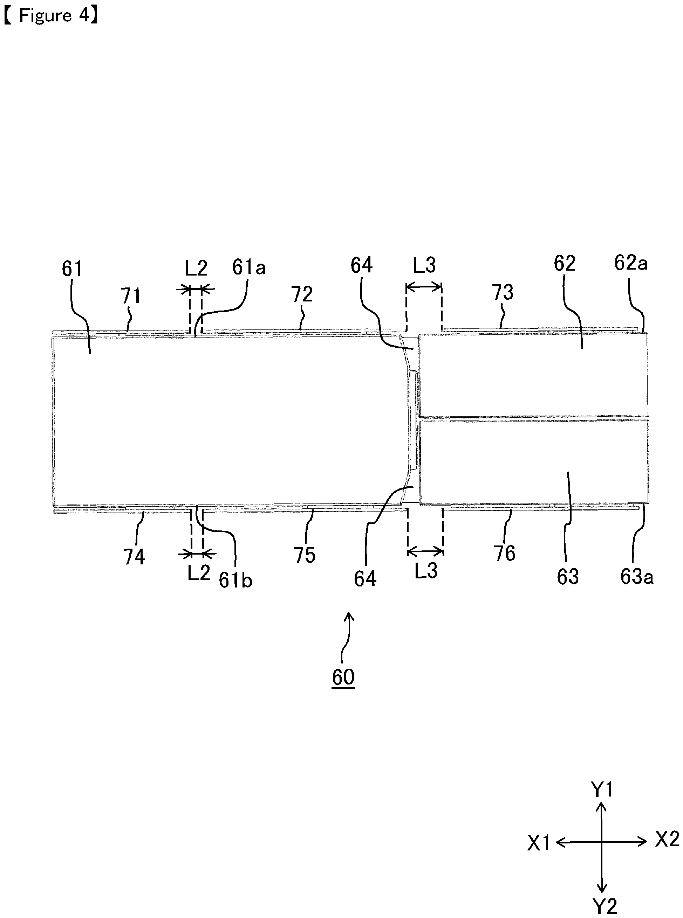

By engaging the engagement portion 42 to any of plate-shaped support portions 71 to 76 (see FIG. 4) provided along the outer periphery of the table portions 61 to 63 of the bed 60, the robot 10 is placed at a desired position in accordance with the surgical procedure, the surgical site, the body shape of the patient, and the like. Thus, the engagement portion 42 as a part of the attachment member allows the main body portion, which is composed of the pedestal unit 30 and the base unit 40, to be detachably attached to any of the table portions 61 to 63 of the bed 60 and to be movable in accordance with the surgical procedure or the like in the front-rear direction (Y1-Y2 direction) or the left-right direction (X1-X2 direction), that is, in a second direction different from the first direction (Z1-Z2 direction) perpendicular to the table portions 61 to 63 of the bed 60.

As illustrated in FIG. 4, the bed 60 includes a first table portion 61 on the left side (X1 side), a second table portion 62 located on the far side on the right side, and a third table portion 63 located on the near side on the right side (see FIG. 1). The three table portions 61 to 63 each have an approximately rectangular shape in a plan view as viewed from the Z1 side to the Z1 side and are arranged on a base 64 so as to be independently movable in the left-right direction (X1-X2 direction). The base 64 extends in the horizontal plane orthogonal to the vertical direction (Z1-Z2 direction).

The base 64 has a rectangular outer shape along the front-rear direction and the left-right direction in a plan view and is supported, as illustrated in FIG. 1, by a support post portion 65 that extends upward from a basement portion 66 placed on the floor surface.

As illustrated in FIG. 1 or FIG. 4, the plate-shaped support portions 71 to 76 are provided on the outer side surfaces of the base 64. The support portions 71 to 76 have a long plate shape of the same thickness and a predetermined width in the up-down direction and extend long in the left-right direction.

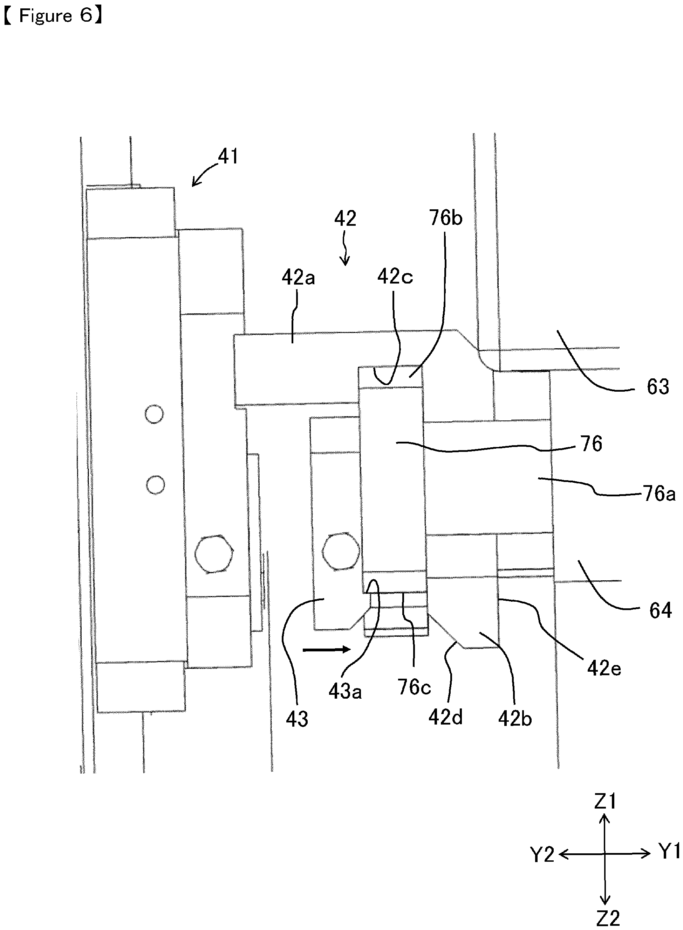

Referring to the sixth support portion 76 as an example, as illustrated in FIG. 6, a connecting portion 76a extending outward from the base 64 along the front-rear direction (Y1-Y2 direction) is fixed to the rear surface of the sixth support portion 76 to support it. In the front-rear direction, the connecting portion 76a has such a length that a rear side surface 42e of the tip engagement portion 42b almost comes into contact with the outer side surface of the third table portion 63 when the tip engagement portion 42b of the engagement portion 42 is hooked to (engaged with) the sixth support portion 76.

As illustrated in FIG. 4, the support portions 71, 72, and 73 are arranged in this order from left to right at the same positions in the front-rear direction, and in the plan view, the support portions 71 and 72 are arranged along a rear surface 61a of the first table portion 61 while the third support portion 73 is arranged along a rear surface 62a of the second table portion 62. The support portions 74, 75, and 76 are arranged in this order from left to right at the same positions in the front-rear direction, and in the plan view, the support portions 74 and 75 are arranged along a front surface 61b of the first table portion 61 while the sixth support portion 76 is arranged along a front surface 63a of the third table portion 63.

The support portions 71, 72, and 73 on the rear side (Y1 side) and the support portions 74, 75, and 76 on the front side (Y2 side) are arranged at positions facing each other in the front-rear direction, and the facing support portions are provided to have the same width (left-right direction). Regarding the distances in the left-right direction (X1-X2 direction), on the rear side, the distance between the first support portion 71 and the second support portion 72 is L2, and the distance between the second support portion 72 and the third support portion 73 is L3, while on the front side, the distance between the fourth support portion 74 and the fifth support portion 75 is L2, and the distance between the fifth support portion 75 and the sixth support portion 76 is L3.

The arrangement, sizes, and distances regarding the support portions are not limited to those illustrated in FIG. 4 or the like. For example, both the front and rear side surfaces of the first table portion 61 may each be provided with one support portion or three or more support portions.

In the example illustrated in FIG. 4, the support portions 71 to 76 extending in the left-right direction are illustrated, but additionally or alternatively to this, support portions extending in the front-rear direction may be provided. In another embodiment, the support portions may be provided on the outer side surfaces of the bed rather than on the base 64.

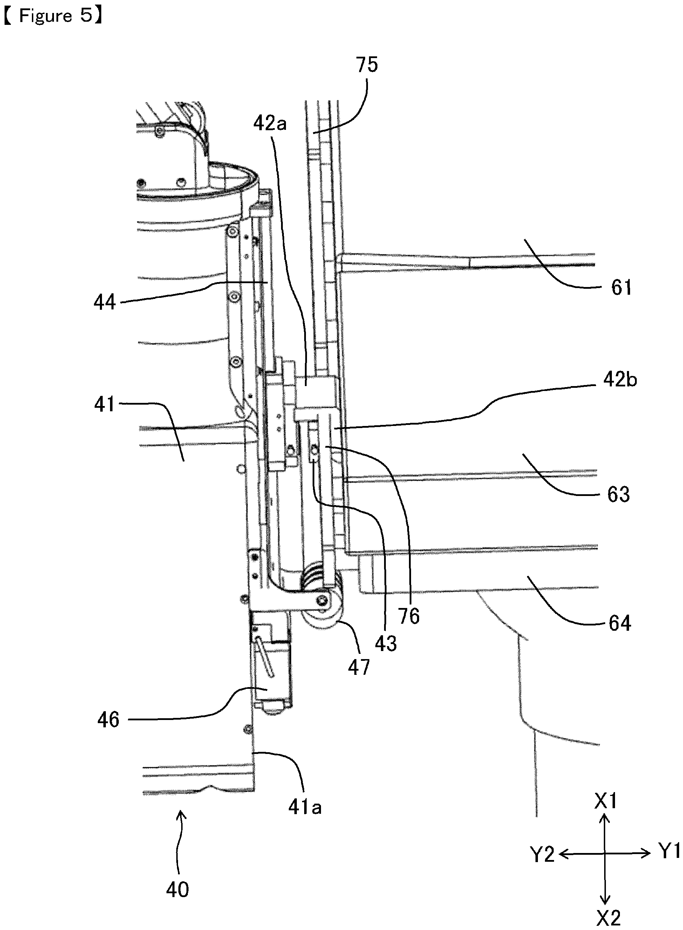

As illustrated in FIGS. 5 and 6, the hold plate 43 of the base unit 40 is arranged so as to extend downward from a mid-position in the front-rear direction of the extending portion 42a of the engagement portion 42. The hold plate 43 is supported by the engagement portion 42 so as to be relatively movable in the front-rear direction. In the process of engaging the base unit 40 with any of the support portions 71 to 76, the hold plate 43 is arranged so that the distance from the tip engagement portion 42b is wider than the thickness of the support portion to be engaged, and after the base unit 40 is engaged with the support portion, the hold plate 43 is moved rearward to adjust the distance from the tip engagement portion 42b to a distance corresponding to the thickness of the engaged support portion, which is thereby clamped in the front-rear direction.

As illustrated in FIG. 6, the extending portion 42a of the engagement portion 42 has a recess 42c at a position at which the sixth support portion 76 to be engaged is engaged. The recess 42c has a length corresponding to the thickness of the sixth support portion 76. The recess 42c is provided to be recessed upward from the lower surface of the extending portion 42a. When the engagement portion 42 is engaged with the sixth support portion 76, the recess 42c houses an upper portion 76b of the sixth support portion 76 and comes into contact with the sixth support portion 76 from the front and rear to determine the position of the sixth support portion 76 in the front-rear direction.

The front portion of the lower end of the tip engagement portion 42b is formed with an inclined surface 42d inclined upward from the rear side (Y1 side) toward the front side (Y2 side). Even if the tip engagement portion 42b hits against the sixth support portion 76 when the engagement portion 42 is engaged with the sixth support portion 76, the tip engagement portion 42b is guided to the rear side of the sixth support portion 42b in accordance with the inclination of the inclined surface 42d, and the engagement of the engagement portion 42 with the sixth support portion 76 can therefore be performed smoothly and reliably.

The rear portion of the lower portion of the hold plate 43 is provided with a protrusion 43a projecting to the rear side. The protrusion 43a is provided at a predetermined position in the up-down direction of the hold plate 43 so as to come into contact with a bottom surface 76c of the sixth support 76 in a state in which the upper portion 76b of the sixth support 76 is housed in the recess 42c. Hence, when the extending portion 42a is engaged with the sixth support portion 76 and the hold plate 43 is then moved to the rear side to reach a position at which the hold plate 43 comes into contact with the front surface of the sixth support portion 76, the protrusion 43a comes into contact with the bottom surface 76c of the sixth support portion 76. Through this operation, the sixth support portion 76 is restricted in the front-rear direction by the tip engagement portion 42b and the hold plate 43 and also restricted in the up-down direction by the protrusion 43a; therefore, the robot 10 comes to a state in which the displacement in the front-rear direction and the up-down direction is restricted to maintain a constant posture of the robot 10 and the robot 10 can move in the left-right direction (X1-X2 direction) along the sixth support portion 76.

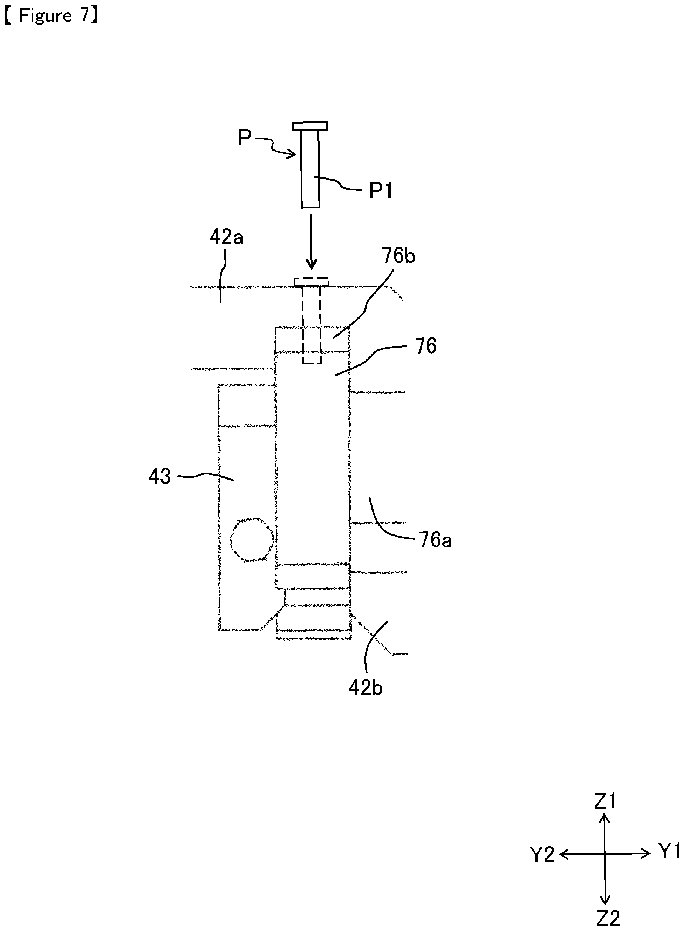

Here, a fixing pin P may be used as a restriction member that restricts the main body portion from moving in a second direction different from the first direction. For example, as illustrated in FIG. 7, the fixing pin P has a shaft portion P1 that penetrates the extending portion 42a in the up-down direction and is further inserted into the sixth support portion 76. When the fixing pin P is used, the movement of the robot 10 in the left-right direction can also be restricted. In FIG. 7, the fixing pin P before being inserted into the sixth support portion 76 is illustrated by a solid line, and the state of being inserted in the sixth support portion 76 is illustrated by a broken line.

As illustrated in FIG. 3, the engagement portion 42 is provided on the rear surface 41a of the housing portion 41 via the linear guide 44 and the ball screw 45 as the vertical drive mechanism. The linear guide 44 is provided from the rear surface 41a of the housing portion 41 to the upper portion of the pedestal unit 30 along the up-down direction, and the ball screw 45 having a screw shaft extending in the up-down direction is placed inside the linear guide 44. The screw shaft of the ball screw 45 is rotated by driving a motor 46 disposed on the lower portion of the rear surface 41a. The above screw shaft is provided with a nut portion (not illustrated) that is displaced up and down by the rotation of the screw shaft. The housing portion 41 fixed to the nut portion is moved up and down relative to the engagement portion 42 while being supported by the linear guide 44, and the robot 10 supported by the base unit 40 is displaced up and down accordingly.

The housing portion 41 is provided with a self-weight compensation spring 47. The self-weight compensation spring 47 is composed, for example, of a tension spring and applies the force which displaces the housing portion 41 upward. By providing the self-weight compensation spring 47, it is possible to suppress the driving force of the motor 46 and efficiently displace the robot 10 up and down.

As illustrated in FIG. 8, the motor 46 is driven based on a control signal from a main body drive control unit 49. On the other hand, a brake 48 (fixing member) is connected to the motor 46, and when the supply of the control signal from the main body drive control unit 49 to the motor 46 is stopped due to a power loss or the like, the brake 48 acts to stop the operation of the ball screw 45, so that the up-down movement of the base unit 40 is stopped to fix the position of the robot 10 in the up-down direction.

Examples of the brake 48 include one that acts on the rotation shaft of the ball screw 45 to restrict the rotation and one that clamps the nut portion, to which the housing portion 41 is fixed, to restrict the up-down movement of the nut portion. Additionally or alternatively, for example, a fixing pin may be inserted into both the housing portion 41 and the engagement portion 42 along the front-rear direction thereby to fix them to each other, so that the up-down movement of the base unit 40 is restricted.

Regarding the operation of the brake 48, for example, as illustrated in FIG. 8, the control signal from the main body drive control unit 49 to the motor 46 is also transmitted to the brake 48, and when the input of the control signal is absent for a predetermined time or more due to a power loss or the like, this situation may be used as a trigger to operate the brake 48. Additionally or alternatively, when there is the control signal from the main body drive control unit 49 to the motor 46, the operation of the brake 48 is restricted, while when the input of the above control signal is absent for a predetermined time or more due to a power loss or the like, the restriction of the operation of the brake 48 may be released to operate the brake 48.

The control signal from the main body drive control unit 49 is also output to a cooperative drive control unit 81. The cooperative drive control unit 81 is connected to the arm drive control unit 82, and control signals given by the arm drive control unit 82 to the first air supply unit 83 and the second air supply unit 84 are also input to the cooperative drive control unit 81.

The cooperative drive control unit 81 coordinates the movement of the base unit 40 in the vertical direction (first direction) and the drive of the arm unit 20. For example, the arm unit 20 is driven in accordance with the vertical position of the base unit 40 to adjust the endoscope 53 to the optimum position and posture for the treatment or the like. When the base unit 40 moves up and down, the posture of the endoscope 53 is changed accordingly to thereby prevent the occurrence of troubles such as contact of the endoscope 53 and the arm unit 20 with the patient.

The cooperative drive control unit 81 stops at least one of the drive of the arm unit 20 and the up-down movement of the base unit 40 at the end of operation, in an emergency, or the like, or drives the arm unit 20 so that the endoscope 53 and the arm unit 20 evacuate to predetermined positions. When stopping the drive of the arm unit 20, the cooperative drive control unit 81 outputs a control signal for adjusting the supply of air from the first air supply unit 83 and the second air supply unit 84 so that the operations of the first pneumatic actuator portions 24b and the second pneumatic actuator portions 26b are fixed. When evacuating the arm unit 20, the cooperative drive control unit 81 outputs a control signal for adjusting the supply of air from the first air supply unit 83 and the second air supply unit 84, which is calculated from the current posture of the arm unit 20 so that the arm unit 20 evacuates to a predetermined position that is preliminarily set.

While the present invention has been described with reference to the above embodiments, the present invention is not limited to the above embodiments and can be improved or modified within the scope of the purpose of improvement or the idea of the present invention.

### [Description of Reference Numerals]

- 10: Robot (surgical robot)
- 20: Arm unit
- 21: Tip holding portion
- 22: Gimbal mechanism portion
- 23: First joint portion
- 24: First arm
- 24a: First rod
- 24b: First pneumatic actuator portion
- 24c: First self-weight compensation portion
- 25: Second joint portion
- 26: Second arm
- 26a: Second rod
- 26b: Second pneumatic actuator portion
- 26c: Second self-weight compensation portion
- 30: Pedestal unit (main body portion)
- 40: Base unit (main body portion)
- 41: Housing portion
- 41a: Rear surface
- 42: Engagement portion
- 42a: Extending portion
- 42b: Tip engagement portion
- 42c: Recess
- 42d: Inclined surface
- 42e: Rear side surface
- 43: Hold plate
- 43a: Protrusion
- 44: Linear guide
- 45: Ball screw
- 46: Motor
- 47: Self-weight compensation spring
- 48: Brake (fixing member)
- 49: Main body drive control unit
- 51: Camera head adapter
- 52: Holder
- 53: Endoscope
- 60: Bed
- 61, 62, 63: Table portion
- 61a, 62a: Rear surface
- 61b: Front surface
- 63b: Front surface
- 64: Base
- 65: Support post portion
- 66: Basement portion
- 71, 72, 73, 74, 75, 76: Support portion
- 76a: Connecting portion
- 76b: Upper portion
- 76c: Bottom surface
- 81: Cooperative drive control unit
- 82: Arm drive control unit
- 83: First air supply unit
- 84: Second air supply unit
- AX1: First axis of rotation
- AX2: Second axis of rotation
- AX3: Third axis o rotation
- P: Fixing pin (restriction member)
- P1: Shaft portion

## Claims

1. A surgical robot comprising:
a main body portion;
an arm unit provided on the main body portion to hold an endoscope;
an attachment member for detachably attaching the main body portion to a table portion of a bed; and
a vertical drive mechanism that allows the main body portion to move along a first direction perpendicular to the table portion,
wherein at least the arm unit is pneumatically driven.

2. The surgical robot according to claim 1, comprising:
an arm drive control unit for driving the arm unit;
a main body drive control unit for moving the main body portion in the first direction; and
a cooperative drive control unit that coordinates the arm unit and the main body portion to allow the arm unit to perform a predetermined operation.

3. The surgical robot according to claim 1 or 2, wherein
the attachment member is configured to be movable with respect to the table portion of the bed along a second direction different from the first direction,
the surgical robot includes a restriction member that restricts the attachment member from moving in the second direction with respect to the table portion of the bed, and
the attachment member is configured to be movable along the second direction when restriction by the restriction member is released.

4. The surgical robot according to any one of claims 1 to 3, comprising
a fixing member that restricts the main body portion from moving along the first direction.

5. The surgical robot according to claim 2, wherein the cooperative drive control unit is configured to evacuate the arm unit to a predetermined position and fix the arm unit at the predetermined position.

6. The surgical robot according to claim 1, wherein the vertical drive mechanism is a ball screw provided on the main body portion, and the attachment member is fixed to a nut portion that is displaced by rotation of a screw shaft of the ball screw.

7. The surgical robot according to claim 6, comprising
a fixing member that restricts the main body portion from moving along the first direction,
wherein the fixing member is a brake that restricts the screw shaft of the ball screw from rotating.

8. The surgical robot according to claim 7, comprising
a main body drive control unit for moving the main body portion in the first direction,
wherein the brake operates when a control signal from the main body drive control unit to a motor for driving the ball screw is absent for a predetermined time.

9. The surgical robot according to claim 1, wherein
the attachment member comprises: an engagement portion that detachably engages the main body portion with the table portion of the bed; and a hold plate that is supported so as to be relatively movable on the engagement portion, and
a support portion arranged around the table portion is clamped by the engagement portion and the hold plate thereby to attach the main body portion to the table portion.

10. The surgical robot according to claim 9, wherein the hold plate includes a protrusion that projects toward the support portion side, and the protrusion and the support portion come into contact with each other thereby to restrict the surgical robot from moving in the first direction.
